# FASCICULE DE BREVET EUROPEEN

(11) **EP 1 736 769 B1**
(45) Date de publication et mention de la délivrance du brevet: **03.07.2013**
(21) Numéro de dépôt: 06010222.5
(22) Date de dépôt: 18.05.2006
(51) Int. Cl.: G01N 33/00, B60H 3/00

(54) **Dispositif et procédé de surveillance et de controle de la qualité de l' air, pour véhicule automobile**
Vorrichtung und Verfahren zur Überwachung und Kontrolle der Luftqualität für Kraftfahrzeuge
Device and procedure for the surveillance and monitoring of the air quality of an automobile

(30) Priorité: 22.06.2005 FR 0506308
(43) Date de publication de la demande: 27.12.2006
(73) Titulaire: VALEO SYSTEMES THERMIQUES, 78321 Le Mesnil St Denis Cedex (FR)
(72) Inventeur: Rede, Laurent, 75014 Paris (FR)
(74) Mandataire: Léveillé, Christophe

(56) Documents cités:
- EP-A- 1 316 799
- EP-A2- 1 398 629
- DE-C1- 19 732 501
- FR-A- 2 785 855
- FR-A- 2 790 557
- US-A- 4 992 965
- US-A- 5 682 317

## Description

L'invention concerne les dispositifs et procédés de surveillance et de contrôle de la qualité de l'air, pour véhicule automobile.

Elle concerne plus particulièrement un dispositif et un procédé de surveillance et de contrôle de la qualité de l'air relié à un capteur de pollution qui délivre une information représentative d'un niveau de pollution dans un flux d'air envoyé dans l'habitacle du véhicule.

Lorsqu'un véhicule traverse une zone polluée, il est souhaitable de disposer du niveau de pollution de l'air dans cette zone, afin d'adapter en conséquence le fonctionnement des équipements du véhicule qui agissent sur la qualité de l'air entrant dans l'habitacle, comme par exemple les dispositifs de purification d'air de type photocatalyse ou plasmacatalyse, les pulseurs d'air ou encore le volet d'entrée d'air du véhicule.

Dans des réalisations existantes, on utilise un capteur de pollution pour adapter le fonctionnement d'un équipement du véhicule, en particulier du volet d'entrée d'air du véhicule.

Classiquement, un volet d'entrée d'air permet de contrôler l'entrée d'air extérieur et l'entrée d'air recirculé d'un boîtier d'entrée d'air pour envoyer dans l'habitacle soit un flux d'air extérieur, soit un flux d'air recirculé, en fonction du confort aérothermique requis dans l'habitacle.

Les capteurs de pollution existants sont adaptés pour détecter la présence d'un polluant dans le flux d'air entrant dans l'habitacle. Ils peuvent être placés en amont ou en aval du filtre à air, par exemple à l'avant du véhicule derrière le pare-chocs ou à proximité du bloc de refroidissement du moteur.

Les capteurs de pollution de l'art antérieur adaptent le fonctionnement de l'équipement du véhicule en fonction du niveau de pollution détecté. Par exemple, ils peuvent faire passer la position du volet d'entrée d'air de véhicule de la position "air extérieur" à la position "air recirculé" en présence d'un niveau de pollution non acceptable. Un exemple d'une telle réalisation est décrit dans le document FR2790557.

Toutefois, l'utilisation d'un capteur de pollution ne permet pas de prendre en compte tous les paramètres du véhicule qui ont une influence sur le niveau de pollution, comme par exemple la vitesse du véhicule. Par ailleurs, le capteur de pollution ne permet pas de prendre en compte l'état de fonctionnement courant de l'équipement du véhicule, par exemple la position du volet d'entrée d'air ou la durée maximale de recirculation d'air, au miment de la détection d'un niveau de pollution non acceptable.

On connaît également des dispositifs de surveillance et de contrôle de la qualité de l'air qui utilisent un capteur de pollution et des machines d'états complexes pour détecter un niveau de pollution et adapter le fonctionnement des équipements du véhicule qui agissent sur la qualité de l'air dans l'habitacle, comme par exemple un volet d'entrée d'air ou un pulseur. Ces machines d'états utilisent non seulement l'information fournie par le capteur de pollution mais aussi d'autres informations spécifiques au véhicule telles que la vitesse du véhicule. Ces dispositifs peuvent adapter le fonctionnement de l'équipement du véhicule en prenant en compte leur état de fonctionnement courant, par exemple le type de distribution d'air courant ou le niveau du pulseur.

Chaque état défini par la machine d'état correspond à un type particulier d'environnement routier pour lequel une stratégie de contrôle de pollution est définie. Partant d'un état donné dans lequel se trouve le véhicule, le véhicule peut soit rester dans le même état, soit passer dans un autre état, soit revenir à l'état précédent, en fonction des données d'entrée.

De tels dispositifs présentent l'inconvénient d'utiliser des machines d'états complexes, qui nécessitent des capacités de traitement et de mémoire importants. Ils déclenchent en outre un nombre élevé de cycles d'ouverture/fermeture du volet d'entrée d'air, ce qui a pour conséquence de provoquer une usure prématurée des actionneurs de ces équipements et une situation d'inconfort thermique, aéraulique et acoustique.

L'invention vient améliorer la situation en proposant un dispositif de surveillance et de contrôle de la qualité de l'air, pour véhicule automobile, relié à un capteur de pollution propre à délivrer une information représentative d'un niveau de pollution dans un flux d'air envoyé dans l'habitacle du véhicule. Le dispositif comporte en outre:
- une mémoire capable de stocker des données relatives à des événements de pollution survenus dans un intervalle de temps précédent de durée prédéfinie, et
- un module de prédiction de pollution propre à déterminer un risque de pollution du flux d'air dans un intervalle de temps futur de durée prédéfinie, à partir d'un nombre d'événements de pollution tiré des données de la mémoire.
- Le module de prédiction de pollution comprend une unité de calcul de probabilité propre à calculer la probabilité d'absence de pollution, dans l'intervalle de temps futur, à partir du nombre d'événements de pollution.

Des caractéristiques optionnelles du dispositif de surveillance et de contrôle de la qualité de l'air selon l'invention, complémentaires ou de substitution, sont énoncées ci-après:
- Le module de prédiction de pollution est apte à comparer la probabilité d'absence de pollution à un seuil de décision sion prédéfini pour déterminer un risque de pollution du flux d'air dans l'intervalle de temps futur.
- Un risque de pollution est déterminé, lorsque la probabilité d'absence de pollution est sensiblement inférieure audit seuil de décision.
- Il comporte en outre au moins un équipement de véhicule agissant sur la qualité de l'air et une unité de gestion apte à adapter le fonctionnement dudit équipement du véhicule en fonction du risque de pollution déterminé par le module de prédiction et de l'information de pollution délivrée par le capteur de pollution.
- L'équipement du véhicule est un volet d'entrée d'air de véhicule ayant une position "d'air extérieur" pour envoyer un flux d'air extérieur dans l'habitacle et une position "d'air recirculé" pour envoyer un flux d'air recirculé dans l'habitacle.
- Le module de prédiction de pollution est apte à transmettre à l'unité de gestion une requête de passage du volet d'entrée d'air dans la position "d'air recirculé", lorsqu'un risque de pollution est déterminé.
- Le module de prédiction de pollution est apte à transmettre à l'unité de gestion une requête de passage du volet d'entrée d'air dans la position "d'air extérieur", en l'absence d'un risque de pollution.
- Il comporte une table de correspondance propre à définir un nombre maximal de mouvements d'ouverture/fermeture du volet en fonction du nombre de kilomètres parcourus par le véhicule depuis sa mise en service, et une unité de mise à jour propre à ajuster la durée de l'intervalle de temps précédent et/ou la valeur du seuil de décision en fonction d'une comparaison entre le nombre de mouvements d'ouverture/fermeture du volet depuis la mise en service du véhicule et ledit nombre maximal de mouvements d'ouverture/fermeture du volet.
- L'unité de mise à jour est apte à autoriser une augmentation de la durée de l'intervalle de temps précédent et/ou de la valeur du seuil de décision, lorsque le nombre de mouvements d'ouverture/fermeture du volet depuis la mise en service du véhicule est supérieur audit nombre maximal de mouvements d'ouverture/fermeture du volet.
- L'unité de mise à jour est apte à diminuer ou maintenir la durée de l'intervalle de temps précédent et/ou la valeur du seuil de décision, lorsque le nombre de mouvements d'ouverture/fermeture du volet depuis la mise en service du véhicule est inférieur ou égal audit nombre maximal de mouvements d'ouverture/fermeture du volet.
- L'unité de gestion de l'équipement du véhicule est apte à déclencher un décompte du temps passé en air recirculé à chaque passage du volet d'entrée d'air dans la position "d'air recirculé", et à faire passer le volet d'entrée d'air dans la position "d'air extérieur", pendant une durée minimale choisie, dès que le temps passé en air recirculé est supérieur ou égal à un seuil choisi représentant le temps maximal de recirculation de l'air.

L'invention propose en outre un procédé de surveillance et de contrôle de la qualité de l'air pour un véhicule automobile muni d'un capteur de pollution propre à délivrer une information représentative d'un niveau de pollution dans un flux d'air envoyé dans l'habitacle du véhicule. Le procédé comporte les étapes consistant à :
- stocker périodiquement des données relatives événements de pollution survenus dans un intervalle de temps précédent de durée prédéfinie,
- déterminer un risque de pollution du flux d'air dans un intervalle de temps futur de durée prédéfinie, à partir d'un nombre d'événements de pollution tiré des données stockées et de l'information de pollution délivrée par le capteur de pollution.

D'autres caractéristiques et avantages de l'invention apparaîtront à l'examen de la description détaillée ci-après et des dessins annexés sur lesquels :
- La figure 1 est un schéma d'un dispositif de surveillance et de contrôle de la qualité de l'air, conforme à l'invention;
- Les figures 2A et 2B sont des schémas d'un boîtier d'entrée d'air classique équipé d'un volet d'entrée d'air et d'un capteur de pollution;
- La figure 3 est un organigramme représentant les différentes étapes mises en oeuvre par le dispositif de surveillance et de contrôle de la qualité de l'air de l'invention ;
- Les figures 4A et 4B sont des organigrammes représentant les différentes étapes mises en oeuvre par le dispositif de surveillance et de contrôle de la qualité de l'air de l'invention pour ajuster le fonctionnement d'un volet d'entrée d'air;
- La figure 5 est un organigramme représentant les différentes étapes mises en oeuvre par le dispositif de surveillance et de contrôle de la qualité de l'air de l'invention pour mettre à jour des paramètres intervenant dans la surveillance de la qualité de l'air; et
- La figure 6 est un diagramme montrant l'évolution de signaux respectifs représentatifs des mouvements d'ouvertures/fermeture d'un volet d'entrée d'air, selon l'art antérieur et selon l'invention.

L'annexe A comporte les équations principales utilisées dans le dispositif et le procédé de l'invention.

Cette annexe est mise à part dans un but de clarification, et pour faciliter les renvois. Elle est partie intégrante de la description, et pourra donc non seulement servir à mieux faire comprendre la présente invention, mais aussi contribuer à sa définition, le cas échéant. Ceci s'applique également en tous points aux dessins.

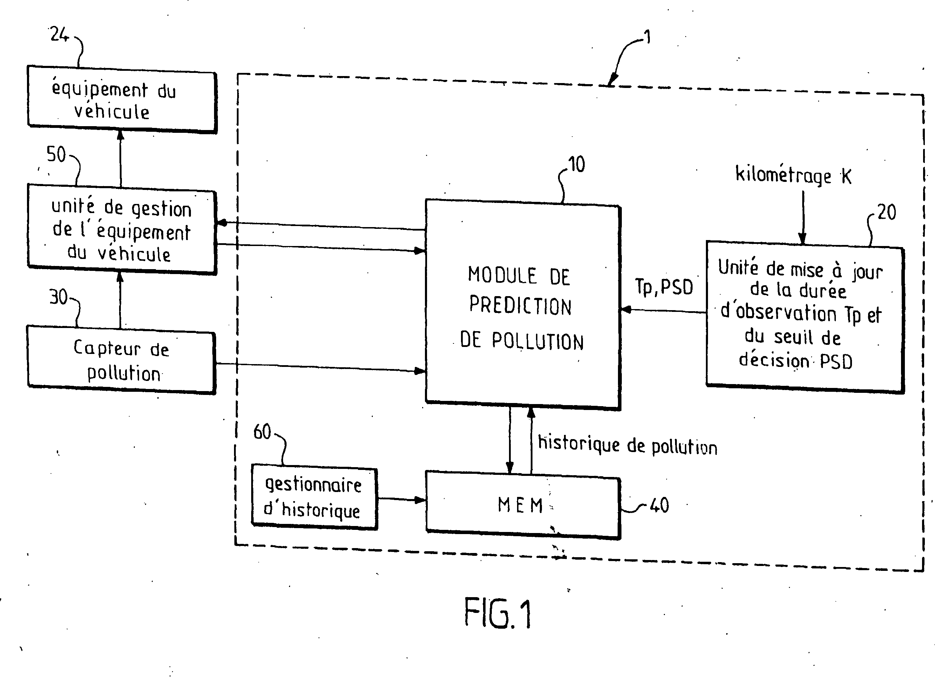

Il est tout d'abord fait référence à la figure 1 qui représente un dispositif de surveillance et de contrôle de la qualité de l'air 1, monté à l'intérieur d'un véhicule automobile. Le dispositif 1 de l'invention est adapté pour surveiller et contrôler la qualité de l'air dans l'habitacle du véhicule.

Le dispositif de,surveillance et de contrôle de la qualité de l'air 1 comporte un module de prédiction de pollution 10 adapté pour prévoir un risque de pollution dans un flux d'air entrant dans l'habitacle du véhicule, dans un intervalle de temps futur.

Le dispositif 1 de l'invention est en outre relié à un capteur de pollution classique 30 qui délivre une information de pollution représentative d'un niveau de pollution dans le flux d'air qui entre dans l'habitacle. L'information de pollution peut être de type numérique ou analogique (tension continue variable, signal PWM, protocole LIN ou CAN, etc.).

Le dispositif de surveillance et de contrôle de la qualité de l'air 1 comporte en outre une mémoire 40 contenant un historique des événements de pollution antérieurs.

La mémoire 40 interagit avec le capteur 30 pour mettre à jour périodiquement l'historique des événements de pollution.

La mémoire 40 interagit de plus avec le module de prédiction 10 pour lui transmettre l'historique de pollution. Le module de prédiction de pollution 10 utilise cet historique de pollution ainsi que l'information de pollution du capteur pour prévoir un risque de pollution dans le flux d'air.

L'invention permet ainsi de prévoir les événements de pollutions susceptibles de se produire dans un intervalle de temps futur, à partir des événements de pollution passés.

A partir de la prévision d'un risque de pollution du flux d'air dans un intervalle de temps futur, le dispositif 1 de l'invention permet d'adapter en conséquence le fonctionnement d'un équipement du véhicule 24 qui agit sur la qualité de l'air, durant cet intervalle de temps, comme par exemple un dispositif de purification d'air de type photocatalyse ou plasmacatalyse, des pulseurs d'air ou encore le volet d'entrée d'air du véhicule.

La suite de la description sera faite en référence à un équipement du véhicule de type volet d'entrée d'air, à titre d'exemple non limitatif.

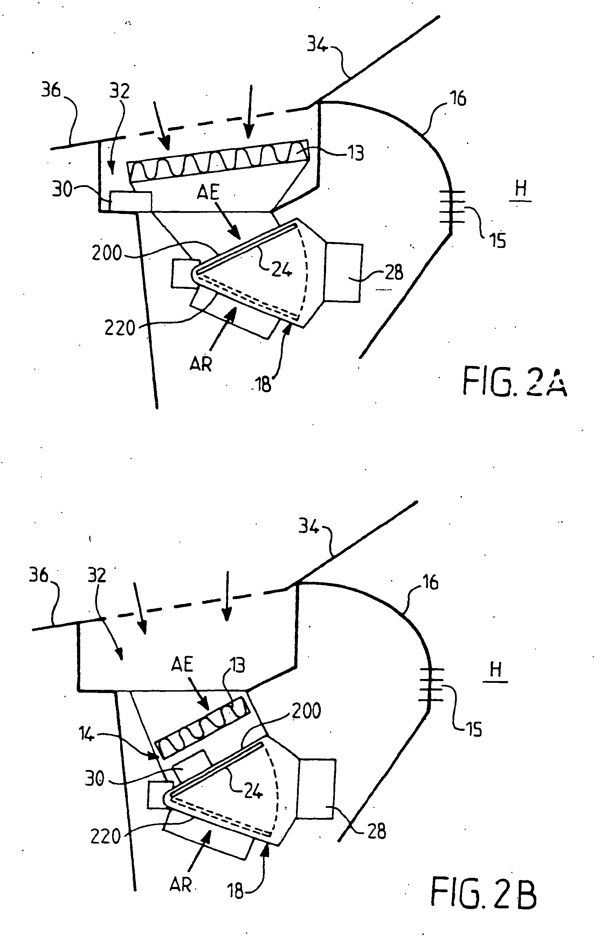

Il est maintenant fait référence aux figures 2A et 2B qui représentent un boîtier d'entrée d'air classique 18 muni d'un capteur de pollution 30.

Le boîtier d'entrée d'air 18 comporte une entrée 200 recevant un flux d'air extérieur AE prélevé hors de l'habitacle H et une entrée 220 recevant un flux d'air recirculé AR prélevé dans l'habitacle H. Le boîtier d'entrée d'air 18 comporte un volet d'entrée d'air 24 propre à pivoter entre une position d'ouverture et une position de fermeture sous l'effet d'une commande, pour contrôler sélectivement les entrées 200 et 220.

Dans la position d'ouverture ou position "d'air extérieur", le volet 24 occupe la position représentée en traits interrompus pour ouvrir l'entrée d'air extérieur 200 et fermer l'entrée d'air recirculé 220.

Dans la position de fermeture ou position "d'air recirculé", le volet 24 occupe la position représentée en traits pleins pour fermer l'entrée d'air extérieur 200 et ouvrir l'entrée d'air recirculé 220.

Le boîtier 18 comporte en outre un pulseur 28 pour envoyer le flux d'air vers des dispositifs de climatisation et/ou de chauffage avant son renvoi dans l'habitacle à travers un aérateur 15 prévu sur la planche de bord 16.

Le capteur de pollution 30 peut être placé en aval d'un filtre à air 13 par rapport à la direction de circulation du flux d'air extérieur AE, comme représenté sur la figure 2A.

En variante, comme représenté sur la figure 2B, le capteur de pollution 30 peut être disposé dans un conduit 14 du boîtier d'entrée d'air 18, qui fait communiquer un séparateur d'eau 32 placé en pied du pare-brise 34 et en haut du capot moteur 36, avec l'entrée d'air 200.

La position du capteur n'est pas limitée à celles décrites en référence aux figures 2A et 2B, à titre d'exemple illustratif.

Le capteur de pollution 30 permet de détecter la présence de polluants, par exemple la présence de monoxyde de carbone contenue dans le flux d'air extérieur.

Le module de prédiction de pollution 10 interagit avec une unité de gestion d'équipement de véhicule 50, qui est en particulier l'unité de gestion de confort du véhicule, qui contrôle le fonctionnement de l'équipement de véhicule 24.

Conformément à l'invention, l'unité de gestion de l'équipement du véhicule 50 adapte le fonctionnement de l'équipement 24 en fonction du risque de pollution déterminé par le module de prédiction.

En complément, l'unité de gestion 50 peut prendre en compte l'information de pollution délivrée par le capteur pour adapter le fonctionnement de l'équipement de véhicule 24.

Après avoir évalué le risque de pollution du flux d'air dans l'intervalle de temps futur, le module de prédiction 10 transmet un signal de commande à l'unité de gestion de l'équipement du véhicule 50.

Lorsque l'équipement du véhicule est un volet d'entrée d'air, ce signal de commande peut comporter une requête de passage du volet d'entrée d'air dans la position "air recirculé", si le risque de pollution déterminé n'est pas acceptable, ou une requête de passage du volet d'entrée d'air dans la position "air extérieur", si le risque de pollution déterminé est acceptable.

L'utilisation sur les véhicules automobiles d'un capteur de pollution 30 fournissant un signal représentatif du niveau de pollution dans l'air de l'habitacle, permet de disposer d'une information de pollution ou de non-pollution en temps réel. Toutefois, cette seule information ne permet pas de prévoir l'évolution de cet état de pollution dans l'intervalle de temps qui suit. Aussi, dans les réalisations de l'art antérieur, le volet d'entrée d'air est commuté selon une fréquence très importante, lorsque des états de pollution successifs sont détectés par le capteur 30. Il en résulte une usure prématurée de l'actionneur du volet d'entrée d'air et une situation d'inconfort thermique, aéraulique et acoustique pour les passagers. Il est alors nécessaire de prévoir des actionneurs à durée de vie relativement longue, et donc coûteux.

L'utilisation de la seule information de pollution délivrée par le capteur ne permet donc pas de prévoir l'apparition d'un événement de pollution dans l'intervalle de temps qui suit pour pouvoir adapter le fonctionnement de l'équipement du véhicule en conséquence sur cet intervalle.

Le dispositif et le procédé de surveillance et de contrôle de la qualité de l'air de l'invention viennent limiter ces inconvénients. En particulier, en fournissant une prévision de la pollution du flux d'air sur l'intervalle de temps qui suit, le dispositif et le procédé de surveillance et de contrôle de l'invention permettent de réduire la fréquence des cycles d'ouverture/fermeture, et de limiter ainsi l'usure de l'actionneur de l'équipement de véhicule. Il n'est alors plus nécessaire de prévoir des actionneurs d'équipement de véhicule à durée de vie très longue.

L'invention propose de surveiller et de contrôler la pollution dans une zone géographique donnée, par exemple une route ou une rue, en prévoyant à un instant donné un risque de pollution future à partir de l'historique des événements de pollution survenus précédemment, par exemple dans les 30 à 45 secondes précédentes.

La Demanderesse a observé en effet que la pollution environnant un véhicule peut être considérée localement et temporairement comme un phénomène aléatoire.

L'utilisation du seul capteur de pollution 30 ou d'une machine d'états de l'art antérieur ne permet pas de prendre en compte le caractère aléatoire de la pollution.

La Demanderesse a déterminé que l'apparition et l'évolution de la pollution dans une zone géographique donnée où évolue le véhicule peuvent être prévues à partir d'une distribution de Poisson.

La distribution de Poisson est utilisée conformément à l'invention pour déterminer la probabilité P(0) de non-apparition d'un événement de pollution de durée choisie Dc, dans une intervalle de temps futur ΔTf de durée choisie Tf, égale à 1 ou 2 secondes par exemple.

Dans une forme de réalisation particulière, la durée Tf de l'intervalle de temps futur est choisie égale à la fréquence d'itération du procédé de surveillance de la qualité de l'air de l'invention, laquelle est avantageusement égale à 1 seconde.

Selon un aspect de l'invention, cette probabilité P(0) peut être déterminée à partir de l'historique des événements de pollution survenus dans un intervalle de temps précédent ΔTp, de durée Tp choisie, compris par exemple entre 30 et 45 secondes.

La structure de l'historique de pollution va maintenant être décrite.

Le dispositif de contrôle de l'invention comporte en outre un gestionnaire d'historique 60, représenté sur la figure 1, propre à mettre à jour l'historique de pollution de la mémoire 40.

Le gestionnaire d'historique 60 est adapté pour échantillonner l'information de pollution délivrée par le capteur de pollution 30, selon une période Tm choisie. En particulier, la période Tm est choisie sensiblement égale à une seconde.

Le gestionnaire d'historique peut stocker chaque événement de pollution sous la forme d'un couple {valeur de l'information de pollution I(ti), instant ti}, l'instant ti correspondant à l'instant où la valeur de l'information de pollution est relevée.

En complément, le gestionnaire d'historique 60 peut comprendre une fonction de conversion propre à convertir chaque valeur stockée de l'information de pollution en un niveau de pollution. Par exemple, un niveau 0 peut être affecté à une valeur de l'information de pollution, si le rapport cyclique variable RC de l'information de pollution à cet instant est inférieur à 30%, un niveau 1 si le rapport est compris entre 30% et 50%, un niveau 2 si le rapport est compris entre 50% et 70%, et un niveau 3 si le rapport est compris entre 70% et 90%. Le gestionnaire d'historique peut alors stocker la valeur de l'information de pollution I(ti) et/ou le niveau de pollution Ni associé, en correspondance avec l'instant ti.

L'historique de pollution stocke ainsi des données relatives à des événements de pollution antérieurs, obtenues à partir de relevés périodiques effectués par le capteur de pollution. Chaque événement de pollution est défini par un instant de pollution ti, une valeur de l'information de pollution I(ti) et/ou un niveau de pollution Ni.

L'historique de pollution comporte donc un enregistrement des événements de pollution passés survenus dans un intervalle de temps précédent Δ Tp.

L'historique de pollution peut avoir une structure de tableau à plusieurs colonnes. Chaque colonne est associé à un événement de pollution relevé par le capteur, défini par l'instant ti d'occurrence de l'événement, la valeur du signal de pollution I(ti) et/ou le niveau de pollution associé Ni.

Le tableau peut se présenter sous la forme d'un tableau à valeurs glissantes de Tp éléments, où Tp est la durée de l'intervalle de temps précédent, encore appelée durée d'observation, pendant laquelle on "observe" les événements de pollutions survenus.

Comme les relevés ont lieu périodiquement selon une période Tm, le nombre d'éléments du tableau Ntab vérifie Ntab = Tp/Tm.

Ainsi, à un instant t0 donné, le module de prédiction 10 peut tirer de l'historique des données relatives à des événements de pollution survenus dans les Tp secondes précédentes.

Le module de prédiction de pollution 10 va maintenant être décrit plus en détail, en référence à l'annexe A.

Le module de prédiction de pollution 10 comporte une unité de calcul de probabilité propre à calculer la probabilité P(0) d'absence de pollution dans un intervalle de temps futur ΔTf de durée Tf. Cette probabilité P(0) correspond à la probabilité de ne voir apparaître aucun événement de pollution de durée choisie Dc dans l'intervalle de temps futur ΔTf.

La probabilité P(0) est obtenue à partir de la probabilité P(m) correspondant à une distribution de Poisson de voir arriver m événements de pollution de durée donnée Dc pendant un intervalle de temps futur ΔTf, conformément à l'équation A1 de l'annexe A

La variable µ correspond à la valeur moyenne ou espérance mathématique de la variable aléatoire m.

Cette variable µ représente la fréquence moyenne d'apparition d'événements de pollution pendant un intervalle de temps futur ΔTf.

La Demanderesse a déterminé que, lorsque la valeur de µ est supérieure à 1, plus d'un événement de pollution par intervalle de temps ΔTf risque de se produire, que lorsque la valeur de µ est égale à 1, des événements de pollution se succédant périodiquement selon une période ΔTf risquent de se produire, et que lorsque la valeur de µ est inférieure à 1, au moins un événement de pollution par intervalle de temps ΔTf risque de se produire.

La probabilité P(0) de ne voir apparaître aucun événement de pollution de durée choisie Dc dans un intervalle de temps futur ΔTf est une fonction de cette variable µ, conformément à l'équation A1 de l'annexe A.

Or, la variable µ peut être exprimée en fonction du nombre Nb d'événements de pollution de durée Dc survenus pendant un intervalle de temps précédent ΔTp, de la variable Dc, de la durée Tf de l'intervalle de temps futur ΔTf, et de la durée Tp de l'intervalle de temps précédent ΔTp, conformément à l'équation A2 de l'annexe A.

La Demanderesse a donc déterminé que la probabilité P(0) de ne voir apparaître aucun événement de pollution de durée choisie Dc dans un intervalle de temps futur ΔTf peut être déterminée à partir du nombre Nb d'événements de pollution de durée Dc survenus pendant un intervalle de temps précédent ΔTp, de la durée choisie Dc du créneau de pollution, de la durée Tf de l'intervalle de temps futur ΔTf, et de la durée Tp de l'intervalle de temps ΔTp, conformément à l'équation A3 de l'annexe A.

Selon un aspect de l'invention, le nombre Nb d'événements de pollution de durée Dc survenus pendant un intervalle de temps précédent ΔTp est tiré de l'historique de pollution stocké dans la mémoire 40.

Pour estimer le nombre Nb d'événements de pollution, le dispositif de contrôle peut comporter une fonction de pondération qui utilise les données de niveau de pollution Ni stockées dans l'historique ou tirés de l'historique pour pondérer chaque événement de pollution associé. Plus précisément, à un événement de pollution de niveau donné, la fonction de pondération peut associer fictivement une pluralité d'événements de pollution en fonction du niveau correspondant. Par exemple, trois événements de pollution pourront être associés à un événement de pollution de niveau 3, deux événements de pollution pourront être associés à un événement de pollution de niveau 2, etc.

La période d'itération Tm du procédé de surveillance de l'invention peut être prise égale à une seconde. La longueur Ntab du tableau à valeurs glissantes est alors égale à la longueur Tp de l'intervalle de temps précédent Δ Tp, avec Ntab = Tp/Tm.

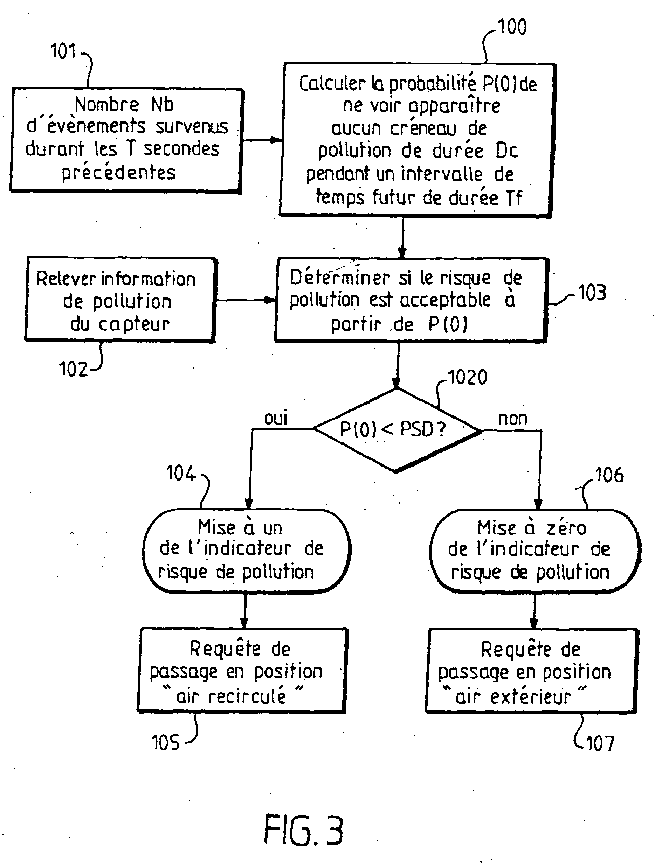

Il est maintenant fait référence à la figure 3 qui est un algorithme illustrant le procédé de surveillance et de contrôle de la qualité de l'air de l'invention.

A chaque itération du procédé de surveillance de la qualité de l'air, les étapes 100 à 106 de la figure 3 sont réalisées.

A l'étape 100, le dispositif calcule la probabilité P(0) de ne voir apparaître aucun créneau de pollution de durée fixée Dc dans un intervalle de temps futur ΔTf de durée prédéfinie, en mettant en oeuvre l'équation A.3 de l'annexe A. En particulier, la durée Tf de l'intervalle de temps futur ΔTf peut être fixée entre 1 et 2 secondes. La probabilité P(0) correspondra alors à la probabilité de ne voir apparaître aucun créneau de pollution de durée Dc, dans les 1 à 2 secondes à venir.

Pour calculer cette probabilité P(0), le dispositif détermine, dans une étape 101, le nombre Nb d'événements de pollution survenus dans un intervalle de temps précédent Δ Tp à partir des données contenues dans l'historique de pollution de la mémoire 40. La durée Tp de l'intervalle de temps précédent peut notamment être comprise entre 30 et 45 secondes. Dans cette forme de réalisation, le dispositif part donc des événements de pollution survenus dans les 30 à 45 secondes précédentes pour évaluer le risque de pollution dans les 1 à 2 secondes à venir, à un instant donné.

Dans une étape ultérieure 103, le dispositif détermine si le risque de pollution représenté par la probabilité P(0) est acceptable.

Pour déterminer si le risque de pollution représenté par la probabilité P(0) est acceptable, le dispositif vérifie à l'étape 1020, si la valeur de la probabilité P(0) de ne voir apparaître aucun créneau de pollution de durée Dc dans l'intervalle de temps futur ΔTf est inférieure à un seuil de décision PSD. Par exemple, le seuil de décision PSD peut être pris égal à 90%. Le seuil de décision PSD peut être choisi en fonction de divers critères, tels que la durée de vie de l'actionneur du volet (en nombre de cycles maximal), le niveau de pollution maximal dans l'habitacle, ou encore le confort acoustique requis dans l'habitacle.

Si la valeur P(0) est supérieure ou égale au seuil de décision PSD, le risque de pollution est acceptable. Le module de prédiction peut alors mettre un indicateur de risque de pollution à la valeur "zéro", à l'étape 104, pour indiquer une absence de risque de pollution.

Le module de prédiction de pollution 10 peut alors transmettre un signal de commande comportant une requête de passage en position "air extérieur" à l'unité de gestion de l'équipement du véhicule 50, qui est en particulier l'unité de gestion de confort de l'habitacle, à l'étape 105. Cette requête indique qu'un passage du volet d'entrée d'air dans la position "air extérieur" est possible.

En revanche, si la valeur P(0) est inférieure au seuil de décision PSD, le risque de pollution n'est pas acceptable. Le module de prédiction peut alors mettre l'indicateur de risque de pollution à la valeur "un", à l'étape 106, pour indiquer la présence d'un risque de pollution.

Le volet d'entrée d'air 24 doit alors passer dans la position "air recirculé" pour empêcher une entrée d'air pollué. Le module de prédiction émet alors un signal de commande comportant une requête de passage en position "air recirculé" à l'unité de gestion de l'équipement du véhicule 50, à l'étape 107.

En complément, le dispositif de l'invention peut prendre en compte la valeur de l'information de pollution du capteur 30, obtenue dans une étape 102, pour initialiser les paramètres.

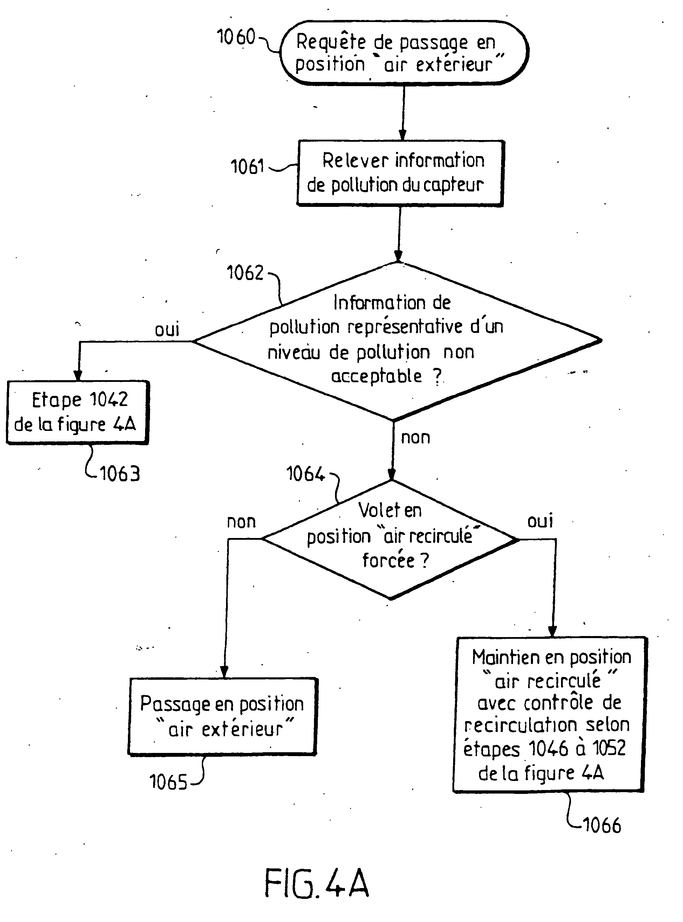

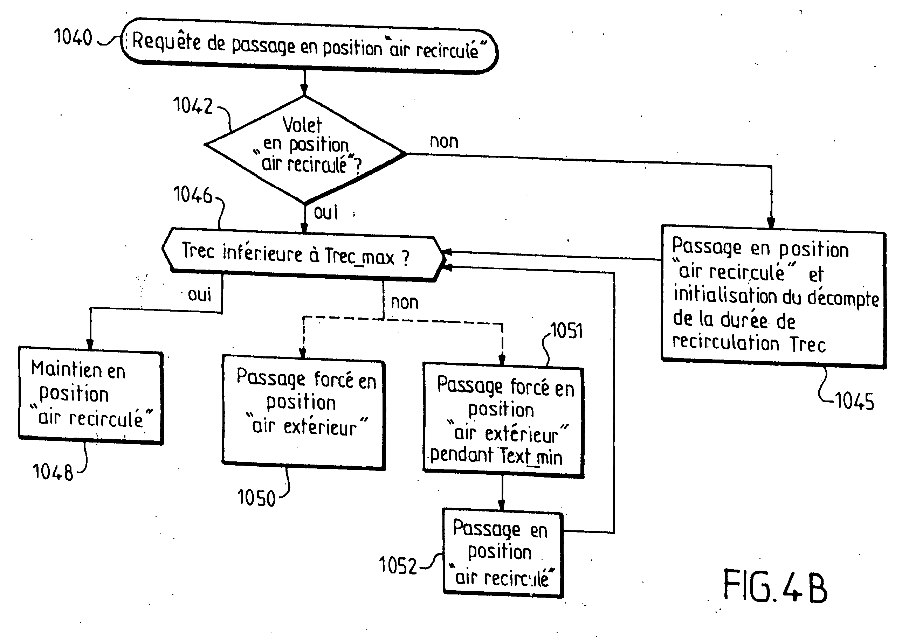

Les requêtes de passage en position "air extérieur" ou en position "air recirculé" sont soumises à l'unité de gestion 50 qui met en oeuvre les organigrammes des figures 4A et 4B pour décider du passage effectif dans la position "air extérieur" ou dans la position "air recirculé" du volet d'entrée d'air 24.

Il est tout d'abord fait référence à l'organigramme de la figure 4A.

Comme indiqué précédemment, lorsque aucun risque de pollution du flux d'air n'est déterminé par le module de prédiction, une requête de passage dans la position "air extérieur" est envoyée à l'unité de gestion du volet d'entrée d'air 50, à l'étape 1060.

A l'étape 1061, l'unité de gestion de l'équipement du véhicule 50 reçoit par ailleurs la valeur de l'information délivrée par le capteur de pollution 30.

A l'étape 1062, l'unité de gestion détermine ensuite si le niveau de pollution de l'information de pollution est inacceptable.

A l'étape 1063, lorsqu'il est déterminé que le niveau de l'information de pollution n'est pas acceptable, l'unité de gestion de véhicule 50 fait passer le volet d'entrée d'air dans la position "air recirculé", au lieu de le faire passer dans la position "air extérieur", pour garantir le confort des passagers. Le procédé de passage du volet d'entrée d'air dans la position "air recirculé" est décrit ci-après en référence aux étapes 1042 à 1052 de la figure 4B.

En privilégiant l'information de pollution du capteur dans le cas où aucun risque de pollution n'est déterminé par le module de prédiction 10, le dispositif de l'invention permet de garantir une bonne qualité de l'air dans l'habitacle.

Lorsque le niveau de l'information de pollution est acceptable, l'unité de gestion de véhicule 50 détermine, à l'étape 1064, si le volet d'entrée d'air 24 se trouve dans la position "air recirculé" forcée, par exemple suite à une commande manuelle d'un passager.

Lorsqu'il est déterminé que le volet d'entrée d'air 24 est dans cette position "d'air recirculé" forcée, l'unité de gestion maintient cette position, à l'étape 1066, en appliquant le procédé de contrôle de recirculation décrit dans les étapes 1046 à 1052 de la figure 4B.

En revanche, lorsque le volet d'entrée d'air 24 n'est pas en position "d'air recirculé" forcée, l'unité de gestion fait passer le volet d'entrée d'air dans la position "d'air extérieur".

Il est maintenant fait référence à l'organigramme de la figure 4B.

Lorsque l'unité de gestion 50 reçoit une requête de passage dans la position "d'air recirculé" suite à la détermination d'un risque de pollution, à l'étape 1040, elle détermine si le volet d'entrée d'air est déjà dans la position "air recirculé" à l'étape 1042, par exemple suite à une commande manuelle d'un passager.

Si le volet d'entrée d'air n'est pas en position "air recirculé", l'unité de gestion fait passer le volet d'entrée d'air dans la position "air recirculé", à l'étape 1045. Elle déclenche également le décompte du temps passé en air recirculé Trec pour effectuer un procédé de contrôle de recirculation.

En effet, la recirculation de l'air peut, si elle est prolongée, provoquer un phénomène de condensation dans l'habitacle qui nuit à la visibilité et au confort des passagers. C'est pourquoi, il convient de limiter la durée de recirculation de l'air. Ainsi, il peut être prévu une durée maximale de recirculation de l'air Trec_max choisie qui, si elle est dépassée, provoque le passage forcé du volet d'entrée d'air dans la position "air extérieur".

L'unité de gestion du volet d'entrée d'air 50 prend en compte cette limitation pour décider ou non d'exécuter la requête de passage en position "d'air recirculé " émise par le module de prédiction 10.

Si le volet d'entrée d'air est déjà en position "air recirculé", l'unité de gestion vérifie, à l'étape 1046, si la durée de recirculation ou temps passé en air recirculé Trec est inférieure à la durée maximale de recirculation Trec_max.

La durée de recirculation Trec dépend du nombre d'événements de pollution rencontrés et de leur intensité.

Cette durée de recirculation Trec dépend en particulier de la durée d'observation Tp et du seuil de décision PSD.

Si le temps passé en air recirculé Trec est inférieur à la durée maximale de recirculation Trec_max, l'unité de gestion 50 maintient le volet d'entrée d'air dans la position "air recirculé", à l'étape 1048.

Si le temps Trec est supérieur à la durée maximale de recirculation Trec_max, l'air doit être renouvelé.

L'unité de gestion 50 peut alors faire passer le volet d'entrée d'air dans la position "air extérieur", à l'étape 1050, et arrêter le décompte du temps passé en air recirculé.

En variante, l'unité de gestion 50 peut faire passer le volet d'entrée d'air dans la position "air extérieur" pendant un temps minimum Text_min pour renouveler l'air, dans une étape 1051. Un décompte du temps de renouvellement Text_min peut être déclenché pour contrôler le renouvellement d'air. Lorsque le temps Text_min a expiré, l'unité de gestion 50 peut alors ramener le volet d'entrée d'air dans la position "air recirculé", après avoir déclenché le décompte du temps de recirculation d'air, dans une étape 1052. Ce temps minimum Text_min est choisi de manière à être suffisamment long pour garantir le renouvellement de l'air et suffisamment court pour ne pas faire entrer une quantité d'air pollué trop importante.

En complément, l'unité de contrôle de pollution peut comporter une unité de mise à jour 20, adaptée pour ajuster la durée d'observation Tp pendant laquelle on mémorise les événements de pollution et/ou le seuil de décision PSD, comme représenté sur la figure 1.

L'unité de mise à jour 20 utilise des informations relatives au nombre réel Nr de mouvements d'ouverture/fermeture du volet d'entrée d'air depuis sa mise en service, au nombre de kilomètres k parcourus depuis la mise en service du véhicule, à la valeur nominale de la durée d'observation Tp et/ou à la valeur nominale du seuil de décision PSD.

L'unité de mise à jour 20 interagit avec une table de correspondance qui fournit le nombre maximal Nt de mouvements d'ouverture/fermeture que peut effectuer lé volet en fonction du nombre de kilomètres k parcourus par le véhicule depuis sa mise en service. Le nombre maximal Nt est ensuite comparé au nombre réel Nr de mouvements d'ouverture/fermeture du volet d'entrée d'air. La durée d'observation Tp et/ou le seuil de décision PSD sont mis à jour en fonction du résultat de la comparaison.

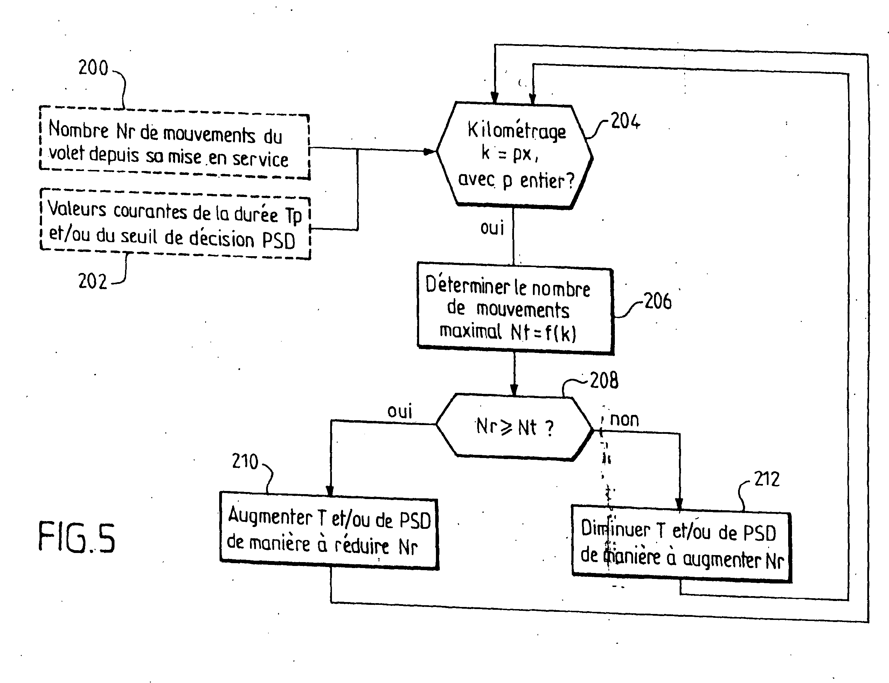

Le procédé de mise à jour de la durée d'observation Tp et/ou du seuil de décision PSD va être décrit plus en détail en référence à la figure 5.

L'unité de mise à jour détermine le nombre réel Nr de mouvements d'ouverture/fermeture du volet depuis la mise en service du véhicule, à l'étape 200. Ce nombre Nr peut être notamment fourni par le calculateur de climatisation du véhicule.

A l'étape 202, elle détermine les valeurs nominales de la durée d'observation Tp et/ou du seuil de décision PSD. Les étapes 200 et 202 peuvent être réalisées successivement dans n'importe quel ordre ou simultanément.

L'unité de mise à jour 20 détermine ensuite le kilométrage k du véhicule depuis sa mise en service et exécute les étapes 206 à 212, périodiquement, lorsque le kilométrage k est un multiple d'une période x (k = px avec p entier).

A l'étape 206, l'unité de mise à jour détermine le nombre maximal Nt de mouvements du volet d'entrée d'air à partir de la valeur du kilométrage k. Le nombre maximal Nt est fourni par la table de correspondance. La table comporte une entrée propre à recevoir un kilométrage et une sortie propre à délivrer le nombre maximal Nt correspondant.

A l'étape 208, l'unité de mise à jour vérifie si le nombre réel Nr de mouvements est supérieur ou égal au nombre maximal Nt de mouvements.

Lorsque le nombre réel Nr de mouvements est supérieur ou égal au nombre maximal Nt de mouvements, l'unité de mise à jour augmente la durée d'observation Tp et/ou le seuil de décision PSD de manière à réduire le nombre Nr de mouvements d'ouverture et de fermeture du volet d'entrée d'air, à l'étape 210.

Lorsque le nombre réel Nr de mouvements est inférieur au nombre maximal Nt de mouvements, l'unité de mise à jour diminue ou maintient la durée d'observation Tp et/ou le seuil de décision PSD de manière à autoriser un nombre Nr de mouvements d'ouverture et de fermeture du volet d'entrée d'air plus important, à l'étape 212.

Pour éviter l'embuage de l'habitacle, l'unité de mise à jour peut utiliser des informations supplémentaires pour mettre à jour la durée d'observation Tp et/ou le seuil de décision PSD, en particulier des informations relatives à l'hygrométrie, à la température de l'air extérieur, ou encore à la température de l'air intérieur.

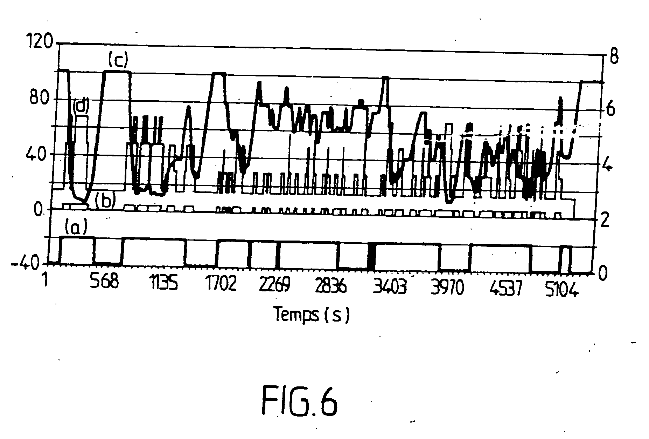

La figure 6 est un diagramme montrant l'évolution d'un signal (b) représentatif des mouvements d'ouvertures/fermetures du volet d'entrée d'air, selon une réalisation de l'art antérieur de type machine d'états, d'un signal (a) représentatif des mouvements d'ouvertures/fermetures du volet d'entrée d'air selon l'invention, d'un signal (c) représentatif de la probabilité P(0) de non-apparition d'un créneau de pollution de durée Dc dans un intervalle de temps futur ΔTf , et d'un signal (d) représentatif du rapport cyclique variable RC de l'information du capteur de pollution.

Le diagramme de la figure 6 est obtenu dans les conditions particulières suivantes :
- durée de l'intervalle de temps futur Tf = 1 seconde, et
- durée de l'intervalle de temps précédent Tp = 30 secondes.

On observe que la fréquence du signal (b) selon l'art antérieur est supérieure à la fréquence du signal (a) selon l'invention. Il a été observé que, dans ces conditions, la fréquence du signal (b) de l'art antérieur peut être sensiblement égale à deux fois la fréquence du signal (a) de l'invention. L'invention permet donc de réduire significativement le nombre de cycles d'ouverture/fermeture du volet d'entrée d'air, tout en garantissant une surveillance et un contrôle efficace de la pollution. Il en résulte une usure de l'actionneur de l'équipement du véhicule 24 moins rapide, ainsi qu'un meilleur confort acoustique et thermique pour les passagers.

L'invention a été décrite en référence à un équipement de véhicule de type volet d'entrée d'air, à titre d'exemple non limitatif. Toutefois, elle est adaptée à une utilisation pour d'autres types d'équipements de véhicule qui agissent sur la qualité de l'air, en particulier pour tout type d'équipement du véhicule qui reçoit un flux d'air extérieur et qui peut être activé/désactivé par une commande, tel qu'un dispositif de purification d'air de type photocatalyse ou plasmacatalyse, ou encore un pulseur d'air.

L'invention n'est pas non plus limitée au contrôle d'un seul équipement du véhicule. Elle peut être utilisée pour contrôler simultanément plusieurs équipements de véhicule de nature distincte.

### Annexe A

**A1.** P(m) = µ^{m}/m! * exp^{(-µ)}
**A2.** µ = (Nb/Dc) * Tf/Tp
**A3.** P(0)= exp^{(-(Nb/Dc)*Tf/Tp)}

## Revendications

1. Dispositif de surveillance et de contrôle de la qualité de l'air, pour véhicule automobile, relié à un capteur de pollution (30) propre à délivrer une information représentative d'un niveau de pollution dans un flux d'air envoyé dans l'habitacle du véhicule, le dispositif comportant en outre:
- une mémoire (40) capable de stocker des données relatives à des événements de pollution survenus dans un intervalle de temps précédent de durée prédéfinie (Tp), et
**caractérisé en ce que** le dispositif comporte un module de prédiction de pollution (20) propre à déterminer un risque de pollution du flux d'air dans un intervalle de temps futur de durée prédéfinie (Tf), à partir d'un nombre (Nb) d'événements de pollution tiré des données de la mémoire (40), le module de prédiction de pollution comprenant une unité de calcul de probabilité propre à calculer la probabilité, d'absence de pollution, dans l'intervalle de temps futur (ΔTf), à partir du nombre d'événements de pollution (Nb).

2. Dispositif selon la revendication précédente, **caractérisé en ce qu'**il comporte en outre au moins un équipement de véhicule (24) agissant sur la qualité de l'air et une unité de gestion (50) apte à adapter le fonctionnement dudit équipement du véhicule en fonction du risque de pollution déterminé par le module de prédiction (10) et/ou de l'information de pollution délivrée par le capteur de pollution (30).

3. Dispositif selon la revendication 2, **caractérisé en ce que** l'équipement du véhicule est un volet d'entrée d'air de véhicule (24) ayant une position "d'air extérieur" pour envoyer un flux d'air extérieur dans l'habitacle et une position "d'air recirculé" pour envoyer un flux d'air recirculé dans l'habitacle.

4. Dispositif selon la revendication 3, **caractérisé en ce que** le module de prédiction de pollution (10) est apte à transmettre à l'unité de gestion (50) une requête de passage du volet d'entrée d'air dans la position "d'air recirculé", lorsqu'un risque de pollution est déterminé.

5. Dispositif selon la revendication 3, **caractérisé en ce que** le module de prédiction de pollution (10) est apte à transmettre à l'unité de gestion (50) une requête de passage du volet d'entrée d'air dans la position "d'air extérieur", en l'absence d'un risque de pollution.

6. Dispositif selon l'une des revendications 3 à 5, **caractérisé en ce qu'**il comporte une table de correspondance propre à définir un nombre maximal (Nt) de mouvements d'ouverture/fermeture du volet en fonction du nombre de kilomètres parcourus par le véhicule depuis sa mise en service, et une unité de mise à jour (20) propre à ajuster la durée de l'intervalle de temps précédent (Tp) et/ou la valeur du seuil de décision (PSD) en fonction d'une comparaison entre le nombre (Nr) de mouvements d'ouverture/fermeture du volet depuis la mise en service du véhicule et ledit nombre maximal de mouvements d'ouverture/fermeture du volet (Nt).

7. Dispositif selon la revendication 6, **caractérisé en ce que** l'unité de mise à jour (20) est apte à autoriser une augmentation de la durée de l'intervalle de temps précédent (Tp) et/ou de la valeur du seuil de décision (PSD), lorsque le nombre (Nr) de mouvements d'ouverture/fermeture du volet depuis la mise en service du véhicule est supérieur audit nombre maximal de mouvements d'ouverture/fermeture du volet (Nt).

8. Dispositif selon la revendication 6, **caractérisé en ce que** l'unité de mise à jour (20) est apte à diminuer ou maintenir la durée de l'intervalle de temps précédent et/ou la valeur du seuil de décision (PSD), lorsque le nombre (Nr) de mouvements d'ouverture/fermeture du volet depuis la mise en service du véhicule est inférieur ou égal audit nombre maximal de mouvements d'ouverture/fermeture du volet (Nt).

9. Dispositif selon l'une des revendications 3 à 8, **caractérisé en ce que** l'unité de gestion de l'équipement du véhicule (50) est apte à déclencher un décompte du temps passé en air recirculé (Trec) à chaque passage du volet d'entrée d'air dans la position "d'air recirculé", et à faire passer le volet d'entrée d'air dans la position "d'air extérieur", pendant une durée minimale choisie (Text_min), dès que le temps passé en air recirculé (Trec) est supérieur ou égal à un seuil choisi représentant le temps maximal de recirculation de l'air (Trec_max).

10. Procédé de surveillance et de contrôle de la qualité de l'air pour un véhicule automobile muni d'un capteur de pollution (30) propre à délivrer une information représentative d'un niveau de pollution dans un flux d'air envoyé dans l'habitacle du véhicule, le procédé comportant les étapes consistant à :
a) stocker périodiquement des données relatives événements de pollution survenus dans un intervalle de temps précédent de durée prédéfinie (Tp),
b) déterminer un risque de pollution du flux d'air dans un intervalle de temps futur de durée prédéfinie (Tf), à partir d'un nombre (Nb) d'événements de pollution titré des données stockées,
**caractérisé en ce qu'**il comporte les étapes consistants à :
- comparer une probabilité d'absence de pollution à un seuil de décision prédéfini (PSD) pour déterminer un risque de pollution du flux d'air dans l'intervalle de temps futur,
- déterminer le risque de pollution lorsque cette probabilité d'absence de pollution est sensiblement inférieure audit seuil de décision (PSD).

11. Procédé selon la revendication 10, **caractérisé en ce qu'**on ajuste le fonctionnement d'un équipement de véhicule (24) agissant sur la qualité de l'air en fonction du risque de pollution déterminé à l'étape b) et/ou de l'information de pollution délivrée par le capteur de pollution (30).

## Patentansprüche

1. Vorrichtung zur Überwachung und Kontrolle der Luftqualität für Kraftfahrzeug, die an einen Verschmutzungssensor (30) angeschlossen ist, der geeignet ist, um eine Information zu liefern, die für ein Verschmutzungsniveau in einem Luftstrom, der in die Fahrgastzelle des Fahrzeugs gesendet wird, repräsentativ ist, wobei die Vorrichtung ferner Folgendes aufweist:
- einen Speicher (40), der fähig ist, Daten in Zusammenhang mit Verschmutzungsereignissen zu speichern, die in einem vorhergehenden Zeitintervall mit vordefinierter Dauer (Tp) aufgetreten sind, und
**dadurch gekennzeichnet, dass** die Vorrichtung ein Verschmutzungsvorhersagemodul (20) aufweist, das geeignet ist, um eine Verschmutzungsgefahr des Luftstroms in einem zukünftigen Zeitintervall mit vorbestimmter Dauer (Tf) ausgehend von einer Anzahl (Nb) von Verschmutzungsereignissen, die aus den Daten des Speichers (40) entnommen wird, zu bestimmen, wobei das Verschmutzungsvorhersagemodul eine Wahrscheinlichkeitsrecheneinheit aufweist, die geeignet ist, um die Wahrscheinlichkeit des Fehlens von Verschmutzung in dem zukünftigen Zeitintervall (ΔTf) ausgehend von der Anzahl von Verschmutzungsereignissen (Nb) zu berechnen.

2. Vorrichtung nach dem vorhergehenden Anspruch, **dadurch gekennzeichnet, dass** sie ferner wenigstens eine Fahrzeugausrüstung (24) aufweist, die auf die Luftqualität einwirkt, und eine Verwaltungseinheit (50), die geeignet ist, um das Funktionieren der Ausrüstung des Fahrzeugs in Abhängigkeit von der Verschmutzungsgefahr anzupassen, die von dem Vorhersagemodul (10) und/oder der von dem Verschmutzungssensor (30) gelieferten Verschmutzungsinformation bestimmt wird.

3. Vorrichtung nach Anspruch 2, **dadurch gekennzeichnet, dass** die Ausrüstung des Fahrzeugs eine Fahrzeug-Lufteinlassklappe (24) ist, die eine Position "Außenluft" hat, um einen externen Luftstrom in die Fahrgastzelle zu senden, und eine Position "rezirkulierte Luft", um einen rezirkulierten Luftstrom in die Fahrgastzelle zu senden.

4. Vorrichtung nach Anspruch 3, **dadurch gekennzeichnet, dass** das Verschmutzungsvorhersagemodul (10) geeignet ist, um an die Verwaltungseinheit (50) eine Anfrage für das Übergehen der Lufteinlassklappe in die Position "rezirkulierte Luft" zu übertragen, wenn eine Verschmutzungsgefahr bestimmt wird.

5. Vorrichtung nach Anspruch 3, **dadurch gekennzeichnet, dass** das Verschmutzungsvorhersagemodul (10) geeignet ist, um an die Verwaltungseinheit (50) eine Anfrage für das Übergehen der Lufteinlassklappe in die Position "externe Luft" bei Fehlen einer Verschmutzungsgefahr zu übertragen.

6. Vorrichtung nach einem der Ansprüche 3 bis 5, **dadurch gekennzeichnet, dass** sie eine Entsprechungstabelle aufweist, die geeignet ist, um eine maximale Anzahl (Nt) von Öffnungs-/Schließbewegungen der Klappe in Abhängigkeit von der Anzahl von Kilometern zu definieren, die von dem Fahrzeug seit seiner Inbetriebnahme zurückgelegt wurden, und eine Aktualisierungseinheit (20), die geeignet ist, um die Dauer des vorhergehenden Zeitintervalls (Tp) und/oder den Beschlussfassungsschwellenwert (PSD) in Abhängigkeit von einem Vergleich zwischen der Anzahl (Nr) von Öffnungs-/Schließbewegungen der Klappe seit der Inbetriebnahme des Fahrzeugs und der maximalen Anzahl von Öffnungs-/Schließbewegungen der Klappe (Nt) anzupassen.

7. Vorrichtung nach Anspruch 6, **dadurch gekennzeichnet, dass** die Aktualisierungseinheit (20) geeignet ist, um eine Erhöhung der Dauer des vorhergehenden Zeitintervalls (Tp) und/oder des Beschlussfassungsschwellenwerts (PSD) zu gestatten, wenn die Anzahl (Nr) von Öffnungs-/Schließbewegungen der Klappe seit der Inbetriebnahme des Fahrzeugs größer ist als die maximale Anzahl von Öffnungs-/Schließbewegungen der Klappe (Nt) .

8. Vorrichtung nach Anspruch 6, **dadurch gekennzeichnet, dass** die Aktualisierungseinheit (20) geeignet ist, um die Dauer des vorhergehenden Zeitintervalls und/oder den Beschlussfassungsschwellenwert (PSD) zu verringern oder aufrecht zu erhalten, wenn die Anzahl (Nr) von Öffnungs-/Schließbewegungen der Klappe seit der Inbetriebnahme des Fahrzeugs kleiner oder gleich der maximalen Anzahl von Öffnungs-/Schließbewegungen der Klappe (Nt) ist.

9. Vorrichtung nach einem der Ansprüche 3 bis 8, **dadurch gekennzeichnet, dass** die Verwaltungseinheit der Ausrüstung des Fahrzeugs (50) geeignet ist, um eine Zählung der mit rezirkulierter Luft verbrachten Zeit (Trec) bei jedem Übergang der Lufteinlassklappe in die Position "rezirkulierte Luft" auszulösen, und die Lufteinlassklappe in die Position "externe Luft" während einer ausgewählten Mindestdauer (Text_min) übergehen zu lassen, sobald die in rezirkulierter Luft verbrachte Zeit (Trec) größer oder gleich einem ausgewählten Schwellenwert ist, der die maximale Rezirkulationszeit der Luft (Trec_max) darstellt.

10. Verfahren zum Überwachen und zur Kontrolle der Luftqualität für ein Kraftfahrzeug, das mit einem Verschmutzungssensor (30) versehen ist, der geeignet ist, eine Information zu liefern, die für ein Verschmutzungsniveau in einem Luftstrom, der in die Fahrgastzelle des Fahrzeugs gesendet wird, repräsentativ ist, wobei das Verfahren die Schritte aufweist, bestehend aus:
a) periodischem Speichern der Daten in Zusammenhang mit Verschmutzungsereignissen, die in einem vorhergehenden Zeitintervall mit vordefinierter Dauer (Tp) aufgetreten sind,
b) Bestimmen einer Verschmutzungsgefahr des Luftstroms in einem zukünftigen Zeitintervall mit vordefinierter Dauer (Tf) ausgehend von einer Anzahl (Nb) von Verschmutzungsereignissen, die aus den gespeicherten Daten entnommen wird,
**dadurch gekennzeichnet, dass** es die Schritte aufweist, bestehend aus:
- Vergleichen einer Abwesenheitswahrscheinlichkeit von Verschmutzung mit einem vordefinierten Beschlussfassungsschwellenwert (PSD), um eine Verschmutzungsgefahr des Luftstroms in dem zukünftigen Zeitintervall zu bestimmen,
- Bestimmen der Verschmutzungsgefahr, wenn diese Verschmutzungsabwesenheitswahrscheinlichkeit deutlich niedriger ist als der Beschlussfassungsschwellenwert (PSD).

11. Verfahren nach Anspruch 10, **dadurch gekennzeichnet, dass** man das Funktionieren einer Fahrzeugausrüstung (24), die auf die Luftqualität in Abhängigkeit von der Verschmutzungsgefahr, die im Schritt b) bestimmt wird und/oder von der von dem Verschmutzungssensor (30) gelieferten Verschmutzungsinformation einwirkt, einstellt.

## Claims

1. Device for a motor vehicle for monitoring and controlling air quality connected to a pollution sensor (30) adapted to deliver information representative of a level of pollution in a stream of air directed into the passenger compartment of the vehicle, the device further including a memory (40) capable of storing data relating to pollution events occurring in a previous time interval (Tp) of predefined duration, **characterised in that** the device includes a pollution prediction module (20) adapted to determine a risk of pollution of the air stream in a future time interval (Tf) of predefined duration from a number (Nb) of pollution events drawn from the data in the memory (40), the pollution prediction module comprising a probability calculating unit adapted to calculate the probability of absence of pollution in the future time interval (ΔTf) based on the number (Nb) of pollution events.

2. Device according to the preceding claim, **characterized in that** it further includes at least one vehicle unit (24) acting on air quality and a management unit (50) capable of adapting the operation of said vehicle unit as a function of the risk of pollution determined by the prediction module (10) and/or pollution information delivered by the pollution sensor (30).

3. Device according to Claim 2, **characterized in that** the vehicle unit is a vehicle air inlet flap (24) having an "outside air" position to direct a stream of outside air into the passenger compartment and a "recirculated air" position to direct a stream of recirculated air into the passenger compartment.

4. Device according to Claim 3, **characterized in that** the pollution prediction module (10) is capable of transmitting to the management unit (50) a request to move the air inlet flap into the "recirculated air" position when a risk of pollution is determined.

5. Device according to Claim 3, **characterized in that** the pollution prediction module (10) is capable of transmitting to the management unit (50) a request to move the air inlet flap into the "outside air" position in the absence of a risk of pollution.

6. Device according to any one of claims 3 to 5, **characterized in that** it includes a correspondance table capable of setting a maximum number (Nt) of opening/closing movements of the flap as a function of the number of kilometres travelled by the vehicle since its commissioning and an update unit (20) adapted to adjust the duration of the preceding time interval (Tp) and/or the value of the decision threshold (PDT) as a function of a comparison between the number (Nr) of opening/closing movements of the flap since the commissioning of the vehicle and said maximum number (Nt) of opening/closing movements of the flap.

7. Device according to Claim 6, **characterized in that** the update unit (20) is capable of allowing an increase in the duration of the preceding time interval (Tp) and/or the value of the decision threshold (PDT) when the number (Nr) of opening/closing movements of the flap since the commissioning of the vehicle is greater than said maximum number (Nt) of opening/closing movements of the flap.

8. Device according to Claim 6, **characterized in that** the update unit (20) is adapted to reduce or maintain the duration of the preceding time interval and/or the value of the decision threshold (PDT) when the number (Nr) of opening/closing movements of the flap since the commissioning of the vehicle is less than or equal to said maximum number (Nt) of opening/closing movements of the flap.

9. Device according to any one of claims 3 to 8, **characterized in that** the management unit of the vehicle unit (50) is capable of initiating a count of the time (Trec) spent in the recirculated air mode each time the air inlet flap goes to the "recirculated air" position and of moving the air inlet flap to the "outside air" position for a chosen minimum duration (Text_min) as soon as the time (Trec) spent in the recirculated air mode is greater than or equal to a selected threshold representing the maximum air recirculation time (Trec_max).

10. Method of monitoring and controlling air quality for a motor vehicle provided with a pollution sensor (30) capable of delivering information representative of a level of pollution in a stream of air directed into the passenger compartment of the vehicle, the method including the steps of:
a) periodically storing data relating to pollution events occurring in a previous time interval (Tp) of predefined duration,
b) determining a risk of pollution of the air stream in a future time interval (Tf) of predefined duration from a number (Nb) of pollution events derived from the stored data, **characterized in that** it includes the steps of:
- comparing a probability of absence of pollution to a predefined decision threshold (PDT) to determine a risk of pollution of the air stream in the future time interval,
- determining the risk of pollution when this probability of absence of pollution is substantially less than said decision threshold (PDT).

11. Method according to claim 10, **characterized in that** the operation of a vehicle unit (24) acting on air quality is adjusted as a function of the risk of pollution determined in step b) and/or the pollution information delivered by the pollution sensor (30).
